(19) Europäisches Patentamt
European Patent Office
Office européen des brevets

(11) Veröffentlichungsnummer : **0 101 807**
**B1**

(12) # EUROPÄISCHE PATENTSCHRIFT

(45) Veröffentlichungstag der Patentschrift : **25.03.87**

(51) Int. Cl.⁴ : **C 07 C125/073**, A 61 K 6/08, A 61 K 6/02

(21) Anmeldenummer : **83105946.4**

(22) Anmeldetag : **17.06.83**

(54) **Neue Tetra(meth)acrylsäureverbindungen und deren Verwendung.**

(30) Priorität : **24.06.82 US 391922**

(43) Veröffentlichungstag der Anmeldung : **07.03.84 Patentblatt 84/10**

(45) Bekanntmachung des Hinweises auf die Patenterteilung : **25.03.87 Patentblatt 87/13**

(84) Benannte Vertragsstaaten : **AT BE CH DE FR GB IT LI NL**

(56) Entgegenhaltungen :
**EP-A- 0 044 352**
**GB-A- 2 074 590**
**GB-A- 2 079 297**
**US-A- 3 629 187**

(73) Patentinhaber : **Blendax-Werke R. Schneider GmbH & Co.**
**Rheinallee 88**
**D-6500 Mainz (DE)**

(72) Erfinder : **Orlowski, Jan A., Dr.**
**1304 Rubio Street**
**Altadena California 91001 (US)**
Erfinder : **Wagner, Helmar R.**
**Römerstrasse 62**
**D-6100 Darmstadt-Arheilgen (DE)**
Erfinder : **Butler, David V.**
**2825 E.Cortez Street**
**West Covina California 91791 (US)**

**EP 0 101 807 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, polymerisierbare Tetra(meth)acrylsäureverbindungen und deren Verwendung in Bindemitteln, insbesondere in Dentalmaterialien, sowie ein dentales Restorations- und Füllungsmaterial.

Es existieren bereits zahlreiche polymerisierbare Verbindungen, die mehr als eine Doppelbindung im Molekül aufweisen. Diese Verbindungen werden für vielfältige Anwendungszwecke verwendet, insbesondere auch als Bindemittel zur Herstellung verschiedener Klebstoffe, u. a. auch in der Medizin und Dentalmedizin, bei der Konfektionierung von Knochenzementen, dentalen Restorations- und Füllmassen, dentalen Versiegelungsmaterialien, orthopädischen und orthodontischen Klebstoffen etc..

Es wurde nun gefunden, daß sich eine neue Klasse von Monomeren, die durch die Umsetzung von aliphatischen, aromatischen oder cycloaliphatischen Diisocyanaten mit bestimmten Hydroxylgruppen enthaltenden Di(meth)acrylaten erhalten werden, besonders gut für den Einsatz als Bindemittel und Klebstoffe, vor allem in den aufgezählten Arbeitsgebieten, eignet.

Gegenstand der Erfindung sind somit neue Verbindungen der allgemeinen Formel

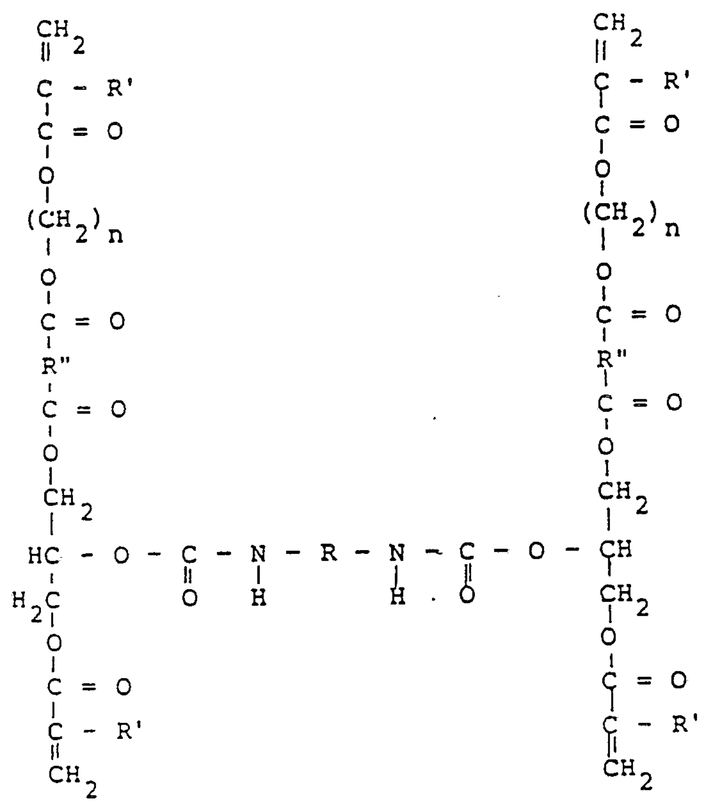

wobei R einen zweiwertigen (ar)aliphatischen, cycloaliphatischen, oder aromatischen Rest mit 4 bis 18 C-Atomen, R′ H o. Methyl, R″ einen —CH=CH—, —$CH_2CH_2$—, —$CH_2CH_2CH_2$—, —$CH_2CH_2CH_2CH_2$—, ggf. substituierten bzw. hydrierten Benzol- oder einen Cyclohexan- oder einen Cis-Norbornenrest, und n 2 oder 3 bedeuten.

Der Rest R steht insbesondere für einen Tetra- oder Hexamethylenrest, einen Phenylen-, Toluylen-, Methylenbisphenyl-, Propylenbisphenyl-, insbesondere Cyclohexan- bzw. Methylen- oder Propylenbiscyclohexylrest.

R′ bedeutet einen Wasserstoffrest oder eine Methylgruppe, wobei die Methacrylverbindung den Vorzug genießt.

R″ bedeutet insbesondere eine —CH=CH-Gruppe (entsprechend Maleinsäure), einen Benzolring (entsprechend Phthalsäure), einen Tetrahydro- oder Hexahydrobenzolring (entsprechend Tetrahydro- oder Hexahydrophthalsäure), einen Cyclohexanring (entsprechend 1,2-Cyclohexandicarbonsäure) oder eine Cis-Norbornengruppe (entsprechend Cis-Norbornendicarbonsäure), jedoch kann beispielsweise auch von Malonsäure-, Bernsteinsäure-, Glutarsäure- oder Adipinsäure-Gruppierungen ausgegangen werden.

Die Herstellung der erfindungsgemäßen polymerisierbaren Verbindungen mit 4 (Meth)Acrylgruppen erfolgt auf folgende Weise :

Ein Anhydrid einer aliphatischen, cycloaliphatischen oder aromatischen Dicarbonsäure wird mit Hydroxyethyl- oder Hydroxypropylacrylat oder -methacrylat, vorzugsweise bei etwa 120 bis 170 °C,

verestert.

Der entstandene Halbester wird nunmehr mit Glycidylacrylat oder -methacrylat zu dem entsprechenden 2-(Meth)Acroylethyl- bzw. 3-(Meth)Acroylpropyl-, 3-(Meth)Acroyl-2-hydroxypropylester umgesetzt.

Dieses Zwischenprodukt wird anschließend durch Reaktion bei etwa 40 bis etwa 100 °C mit einem Diisocyanat in das entsprechende Biscarbamat überführt.

Aus der DE-B 2 357 324 und der DE-A 2 419 887 sowie der US-A 3 425 988 sind zwar ebenfalls bereits (Meth)Acrylsäureester bekannt, die Carbaminsäuregruppierungen im Molekül aufweisen, jedoch handelt es sich bei diesen bekannten Produkten um von den erfindungsgemäßen Monomeren strukturell unterschiedliche Produkte, mit denen die durch die Verwendung der erfindungsgemäßen Zusammensetzungen erzielbaren Vorteile nicht erreicht werden können.

Schließlich beschreibt die GB-A 2 079 297 ein härtbares dentales Füllungsmaterial, das in seiner Harzkomponente Reaktionsprodukte aus Diisocyanaten und Hydroxalkyldiacrylaten und -methacrylaten der allgemeinen Formel

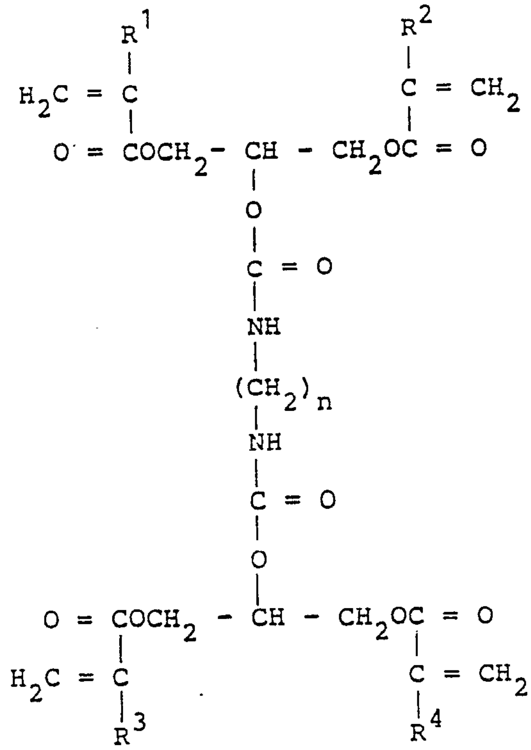

wobei $R^1$, $R^2$, $R^3$ und $R''$ Wasserstoff oder Methylgruppen bedeuten und n eine ganze Zahl von 2 bis 10 darstellt.

Die durch die Mitverwendung solcher Monomerer in gehärteten dentalen Füllungsmaterialien erzielbare Verbesserung der mechanischen Eigenschaften, insbesondere der Härte, bleibt jedoch hinter den Erfordernissen der zahnärztlichen Praxis zurück.

Die erfindungsgemäßen Produkte eignen sich grundsätzlich für alle diejenigen Verwendungszwecke, wo Monomere mit mehreren polymerisierbaren Doppelbindungen im Molekül Einsatz finden können. Wie bereits angedeutet, sind sie besonders geeignet zur Anwendung in der Medizin und Dentalmedizin.

In der Dentalmedizin haben besonders die aus Füllstoffen und polymerisierbaren Verbindungen bestehenden Füllungsmaterialien, die sogenannten « Composites », in den letzten Jahren zunehmende Bedeutung erlangt, da diese Produkte vom Zahnarzt leicht und sicher zu handhaben sind, vom Patienten in der Regel gut und reizlos vertragen werden und die Möglichkeit bieten, von den aus physiologischen Gründen immer wieder kritisierten Amalgam-Füllungsmaterialien abzurücken.

Nachteile der Composites gegenüber den herkömmlichen Füllungsmaterialien sind jedoch ihre Abrasions- und Schrumpfungsanfälligkeit und ihre Wasserabsorption ; besonders durch die mögliche Schrumpfung können zwischen Füllungsrand und ausgefüllter Kavität im Laufe der Zeit sekundärkariöse Erscheinungen auftreten.

Es ist daher das Bestreben der Fachwelt, Composites herzustellen, die nach Möglichkeit keine Schrumpfung aufweisen, eine geringe Wasserabsorption besitzen, gute mechanische Eigenschaften, insbesondere Härte, aufweisen und farbstabil sind.

Diese Eigenschaften lassen sich durch einen möglichst hohen Anteil des Füllermaterials in den Zusammensetzungen erreichen. Die Höchstmenge des einzusetzenden Fülleranteils ist jedoch abhängig von den Eigenschaften der in der Zusammensetzung eingesetzten Monomeren.

Übliche Composites weisen nach ihrer Aushärtung in der Regel folgende mechanische Eigenschaftswerte auf :

| | |
|---|---|
| Wasserabsorption bei 37 °C : | 0,7-1,2 mg/cm² x) |
| Druckfestigkeit : | 30 000 bis 40 000 psi |
| Diametrale Zugfestigkeit : | 3 480 bis 4 200 psi x) |
| Härte (Barcol) : | 98 |
| Farbstabilität : | Farbveränderung nicht erkennbar x) |
| Opazität/Transluzenz-Faktor : | 0,35 bis 0,55 x) |
| Gewichtsverhältnis Füller zu Harz : | 3,5 : 1 bis 4,2 : 1 |

x) bestimmt entsprechend der im « Journal of the American Dental Assoc., Vol. 94 (Juni 1977) » beschriebenen Specification N° 27.

Es wurde nun gefunden, daß man die aufgeführten mechanischen Eigenschaften üblicher, bekannter Composites durch den Einsatz der erfindungsgemäßen Monomeren ganz wesentlich verbessern kann und insbesondere ein wesentlich erhöhtes Verhältnis von Füllstoff zu Harz erreicht werden kann, was wiederum eine ebenso wesentliche Verbesserung der physikalischen Eigenschaften, vor allem der Härte und Abriebfestigkeit, der Füllungen zur Folge hat.

Der Füllstoffanteil in der Gesamtzusammensetzung läßt sich dabei bis auf etwa 90 % steigern.

Gegenstand der vorliegenden Erfindung ist daher auch ein dentales Füllungsmaterial, das gekennzeichnet ist durch einen Anteil zumindest eines der erfindungsgemäßen Monomeren, eines Füllers, eines Polymerisationsinitiators oder Beschleunigers, sowie gegebenenfalls weiterer an sich bekannter Stoffe wie weiterer Monomerer, UV-Absorber, Stabilisatoren, Farbstoffe etc..

Besonders vorteilhaft ist ein dentales Füllungsmaterial, das die folgenden Bestandteile enthält :

5 bis 50 Gew.-% mindestens einer erfindungsgemäßen Tetra(meth)acrylsäureverbindung ;

0,1 bis 5,0 Gew.-% mindestens eines Polymerisationsinitiators und/oder Polymerisationsbeschleunigers ;

50 bis 90 Gew.-% mindestens eines Füllstoffes ;

sowie gegebenenfalls weitere polymerisierbare Verbindungen, UV-Absorber, Farbstoffe, Haftungsverbesserer und sonstige in solchen Mitteln an sich übliche Zusätze.

Bei den eingesetzten Füllungsmaterialien kann es sich um röntgendurchlässige oder röntgenundurchlässige Materialien handeln. Beispiele sind insbesondere die verschiedenen Siliciumdioxid-Modifikationen, wie Glas (pulverisiertes Glas), Borsilicatglas und andere Gläser wie Quarzit, Cristobalit u. ä. für röntgendurchlässige Füllstoffe und Bariumaluminiumsilicat, Lithiumaluminiumsilicat oder Glaskeramik-Füllstoffe, die beispielsweise die Elemente Lanthan oder Zirkonium enthalten, für röntgenundurchlässige Füllstoffe. Geeignete röntgenundurchlässige Füllstoffe sind beispielsweise in den US-A 3 801 344, 3 808 170 und 9 975 203 und der DE-A 2 347 591 beschrieben.

Zur Erhöhung der Haftfestigkeit können diese Füllstoffe auch in bekannter Weise silanisiert sein.

Die Teilchengrößen der eingesetzten Füllstoffe bewegen sich in der Regel zwischen etwa 0,01 und 100 Mikron ; es ist in vielen Fällen möglich und auch sinnvoll, Kombinationen aus Füllstoffen niederer und höherer Teilchengrößen einzusetzen, wobei der bevorzugte Teilchendurchmesser zwischen etwa 0,05 und etwa 50, vorzugsweise etwa 30 Mikron liegt.

Geeignete Füllstoffe sind auch die in der Europäischen Patentanmeldung Nr. 81102198.9 vom 24.3.1981 und in der US-Patentanmeldung Nr. 368,743 vom 15.4.1982 beschriebenen Füllstoffe bzw. Füllstoff-Gemische.

Composites liegen in zwei Modifikationen vor, entweder als zweiphasige Präparate, von denen die eine Phase einen Polymerisationsinitiator, beispielsweise ein Peroxid, und die andere Phase einen Beschleuniger für dieses Peroxid, beispielsweise ein organisches Amin, enthält, wobei das Zusammenbringen beider Phasen unmittelbar vor der Zahnfüllung erfolgt und die Polymerisation in der aufgebohrten, vorzugsweise mit einem Unterfütterungsmaterial versehenen, zu füllenden Kavität eintritt.

Es ist jedoch auch möglich, einphasige Präparate herzustellen, die unter Einwirkung von Licht, beispielsweise UV- oder Laserlicht, polymerisieren und dann selbstverständlich einen Photopolymerisationsinitiator und gegebenenfalls auch einen Beschleuniger dafür enthalten müssen.

Der Einsatz der erfindungsgemäßen Monomeren ist selbstverständlich sowohl in Zwei- als auch in Einphasenpräparaten möglich.

Für Einphasenpräparate kommt in der Regel die Polymerisationsenleitung durch Lichteinfluß in Betracht. Entsprechende Photopolymerisationsinitiatoren sind bekannt, vorzugsweise handelt es sich dabei um Carbonylverbindungen, insbesondere Benzil und Benzilderivate, wie 4,4-Oxydibenzil oder andere Dicarbonylverbindungen, z. B. Diacetyl, 2,3-Pentandion, oder Metallcarbonyle, Chinone und deren Derivate. Der Anteil an Photopolymerisationsinitiator beträgt etwa 0,01 bis etwa 5 Gew.-% der Gesamtzusammensetzung.

Diese einphasigen Präparate enthalten vorzugsweise auch noch sogenannte Polymerisationsbeschleuniger. Dies sind Substanzen, die in Gegenwart von Polymerisationsinitiatoren die Polymerisationsreaktion beschleunigen.

Bekannte Beschleuniger sind beispielsweise verschiedene Amine wie p-Toluidin, Dimethyl-p-toluidin, Trialkylamine, Polyamine wie N,N,N',N'-Tetraalkylalkylendiamine und Sulfimide, vorzugsweise in einer Menge von etwa 0,01 bis etwa 5 Gew.-% der Gesamtzusammensetzung.

Soll das die erfindungsgemäßen Monomeren enthaltende Dentalfüllungsmaterial nicht lichthärtbar sein und in zwei bis zur Anwendung voneinander getrennt gehaltenen Phasen vorliegen, so enthält eine dieser Mischungen in der Regel einen Polymerisationsinitiator. Dies sind zumeist Peroxide, die bei der Auslösung der Polymerisation unter Radikalbildung zerfallen. Geeignete Peroxide sind beispielsweise Arylperoxide wie Benzoylperoxid, Cumolhydroperoxid, Harnstoffperoxid, tert.-Butylhydroperoxid oder -perbenzoat und Silylperoxide, in Mengen von vorzugsweise etwa 0,01 bis etwa 5, insbesondere etwa 0,5 bis 2,5 Gew.-%.

Enthält die eine Phase des zweiphasigen Mittels einen Polymerisationsinitiator, so wird der anderen Phase zweckmäßigerweise ein Beschleuniger des oben beschriebenen Typs, vorzugsweise ein Amin, zugesetzt.

In diesen erfindungsgemäßen Monomeren enthaltenden Dentalfüllungsmaterialien können zur Verbesserung der Haftfähigkeit zwischen Füllstoff und Harz polymerisierbare Organosiliciumverbindungen mitverwendet werden, beispielsweise Methacryloylalkyltrihydroxysilane oder Methacryloyltrimethoxysilan.

Zusätzlich zu den erfindungsgemäßen Monomeren können in den eingesetzten dentalen Füllungsmaterialien weitere für diesen Zweck bereits vorgeschlagene Monomere mit verwendet werden. Solche Monomere sind beispielsweise Alkandioldimethacrylate wie 1,6-Hexandioldimethacrylat, 1,4-Butandioldimethacrylat oder Tri- oder Tetraethylenglykoldimethacrylat, Bis-(2-methacryloylethyl)-phthalat, -isophthalat oder -terephthalat, Trimethylolpropandi- und trimethacrylat, Reaktionsprodukte aus Diisocyanaten und einfachen Hydroxyalkylmethacrylaten, wie sie beispielsweise in der DE-A 2 312 559 beschrieben sind, Reaktionsprodukte aus Bisphenolen, insbesondere Bisphenol A und Glycidylmethacrylat (Bis-GMA), Addukte aus (Di-)Isocyanaten und 2,2-Propanbis-3-(4-phenoxy)-1,2-hydroxypropan-1-methacrylat nach der US-A 3 629 187 sowie sonstige für diesen Zweck bereits vorgeschlagene polymerisierbare Verbindungen.

Geeignete Monomere sind auch die in der EP-A 44 352 beschriebenen Addukte aus Methacroylalkylethern, -alkoxybenzolen bzw. -alkoxycycloalkanen und Diisocyanaten.

Zur Einstellung eines möglichst naturgetreuen Eindrucks der gefüllten Zahnflächen enthalten Composite-Materialien zuweilen Farbstoffe in geringen Mengen.

Weiterhin ist der Zusatz geringer Mengen an UV-Stabilisatoren möglich. Geeignete Stabilisatoren sind z. B. Hydrochinon, p-Benzochinon, p-Butylhydroxytoluol u. ä..

Die folgenden Beispiele dienen der Erläuterung der Erfindung :

### Beispiel A

a) 98 g Maleinsäureanhydrid und 0,35 g 2,6-Di-tert.-butyl-4-methylphenol werden bei 70 °C in 130 g Hydroxyethylmethacrylat unter Rühren und Verwendung eines Rückflußkühlers gelöst. Die Temperatur des Gemisches wurde auf 130 °C erhöht und 180 g Glydicylmethacrylat stufenweise unter Rühren bei 130-140 °C während einer Stunde zugesetzt. Alle 30 Minuten wurden Säurezahl und Epoxyäquivalent bestimmt. Wenn die Säurezahl 0,5 unterschreitet, wird der Kühler vom Reaktionsgefäß entfernt und Luft durch das Reaktionsgemisch geblasen, bis das Epoxyäquivalent 10,000 überschreitet. Die Reaktion ist nach etwa 5 Stunden beendet ; es wird 2-Methacroylethyl-, 3-Methacroyl-2-hydroxypropyl(maleat),

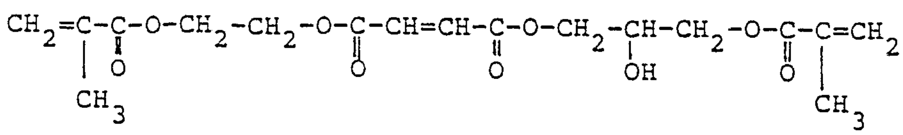

in praktisch quantitativer Ausbeute erhalten.

In analoger Weise lassen sich die entsprechenden Acrylverbindungen herstellen.

b) 92,5 g des nach a) erhaltenen Reaktionsproduktes und 20 g Hexamethylendiisocyanat werden bei 70° in Gegenwart von 0,1 g Dibutylzinndiacetat zusammengerührt. Die Reaktion ist nach etwa 3 Stunden beendet, wenn keine freien Isocyanatgruppen mehr nachweisbar sind.

Das Reaktionsprodukt, eine farblose, hochviskose Masse, wies die folgende Struktur auf :

(Siehe Formel Seite 6 f.)

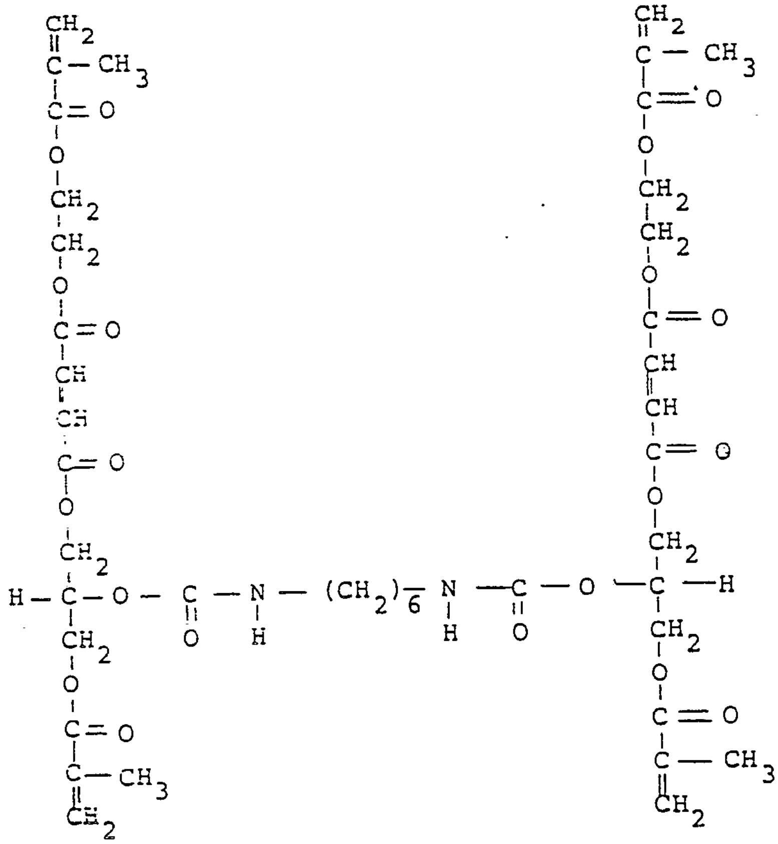

Brechungsindex (100 °C) : 1,470.

In gleicher Weise ließen sich das entsprechende Tetramethylen- und Phenylenbiscarbamat herstellen.

Beispiel B

185 g des nach Beispiel A, a) hergestellten 2-Methacroylethyl-, 3-Methacroyl-2-hydroxypropyl(maleats) wurden mit 60 g Bis (4-isocyanatophenyl)-methan bei 90 °C-95 °C unter Rühren umgesetzt.

Die Reaktion war nach 90 Minuten, als keine freien Isocyanatgruppen im Reaktionsgemisch nachweisbar waren, beendet.

Das Reaktionsprodukt ist eine bei Raumtemperatur halbfeste, farblose Masse, bestehend aus dem Methylenbisphenylcarbamat des 2-Methacroylethyl, 3-Methacroyl-2-hydroxypropyl(maleats) der Struktur

(Siehe Formel Seite 7 f.)

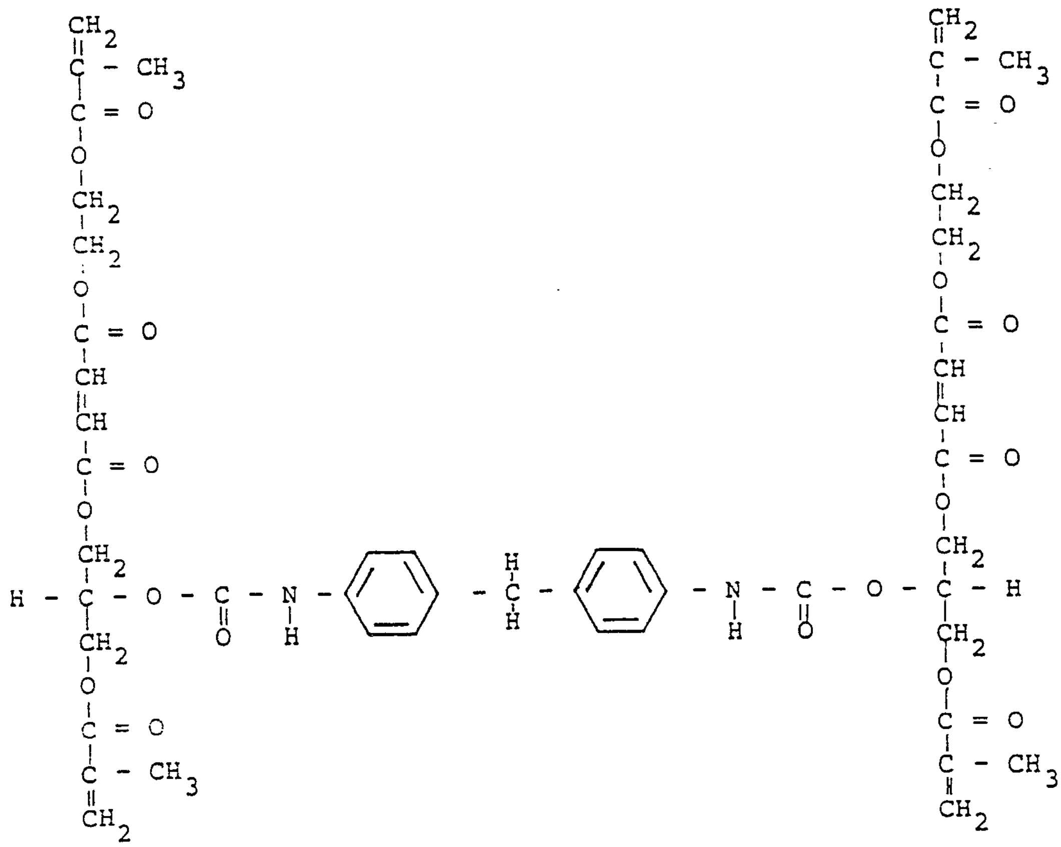

Brechungsindex (100 °C) : 1,513

Auf analoge Weise läßt sich das entsprechende Methylenbiscyclohexylcarbamat herstellen.

Beispiel C

a) 222 g Phthalsäureanhydrid und 0,3 g 2,6-Di-tert.-butyl-4-methylphenol werden in 195 g 2-Hydroxyethylmethacrylat unter Rühren gelöst. Die Temperatur wird am Rückflußkühler auf 135 °C erhöht und stufenweise 270 g Glycidylmethacrylat unter Rühren während 1 Stunde bei 135-145 °C zugesetzt. Alle 30 Minuten wurden die Säurezahl und das Epoxyäquivalent bestimmt. Nach weiteren 2,5-stündigem Rühren lag die Säurezahl bei 0,2 und das Epoxyäquivalent bei 5,000. Der Rückflußkühler wurde entfernt und der nicht reagierte Überschuß an Glycidylmethacrylat durch Luft ausgetrieben, bis ein Epoxyäquivalent von 10,000 erreicht war.

Es wurde 2-Methacroylethyl-, 3-Methacroyl-2-hydroxypropyl(phthalat) der Struktur

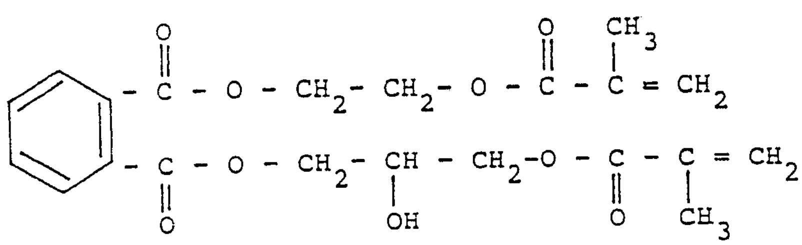

in praktisch quantitativer Ausbeute erhalten.

b) 211 g des nach a) erhaltenen Reaktionsproduktes wurden unter Rühren bei 70 °C mit 90 g Hexamethylendiisocyanat in Gegenwart von 0,25 g Dibutylzinndiacetat vermischt. Nach insgesamt 5 Stunden war die Reaktion beendet, als keine freien Isocyanatgruppen nachweisbar waren.

Das Reaktionsprodukt, eine bei Raumtemperatur farblose, halbfeste Masse, wurde als das Hexamethylenbiscarbamat des 2-Methacroylethyl-, 3-Methacroyl-2-hydroxypropyl(phthalats) der Strukturformel

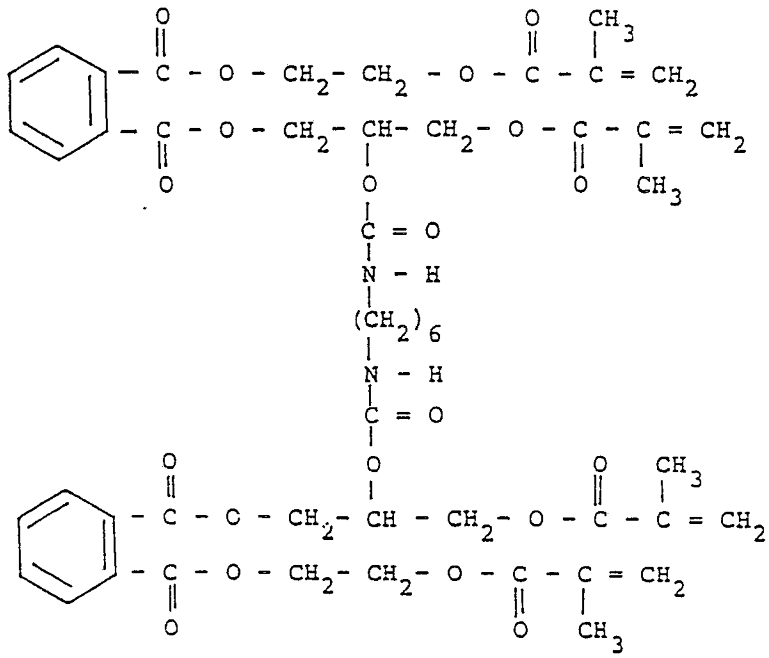

identifiziert.

Brechungsindex (100 °C) : 1,495

In gleicher Weise ließen sich das entsprechende Tetramethylen- und Toluylenbiscarbamat herstellen.

Beispiel D

Entsprechend den in Beispiel C, b) beschriebenen Reaktionsbedingungen wurde das Methylenbisphenylcarbamat des 2-Methacroylethyl-, 3-Methacroyl-2-hydroxypropyl(phthalats) erhalten :

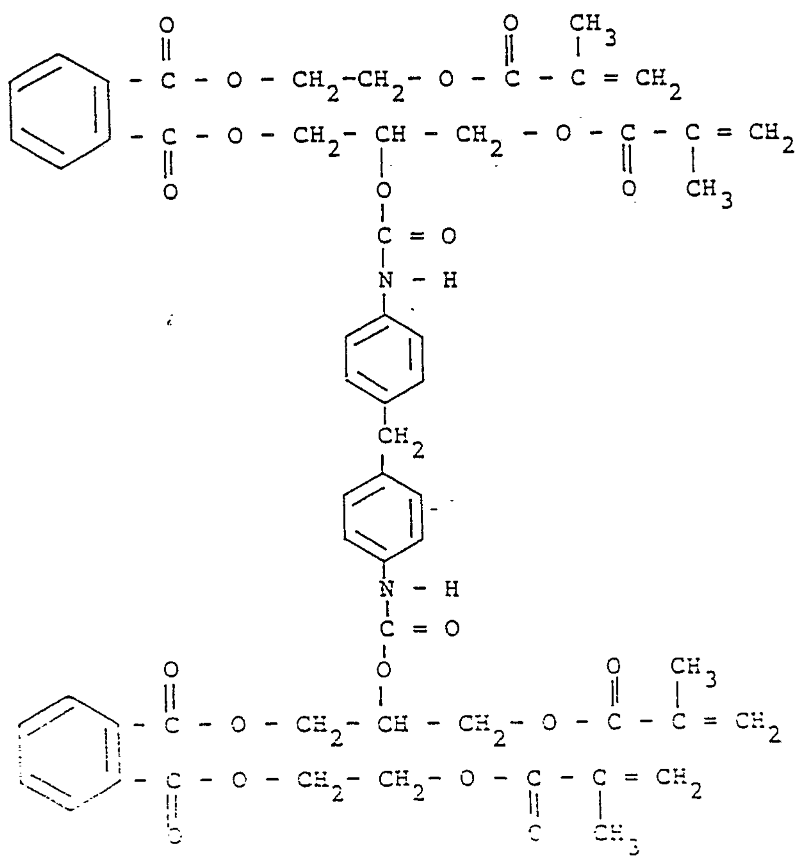

Brechungsindex (100 °C) : 1,495

Analog wurde das Methylenbiscyclohexylcarbamat hergestellt.

Beispiel E

a) Analog dem in Beispiel C beschriebenen Verfahren wurde aus Hydroxyethylmethacrylat und Cyclohexan-1,2-dicarbonsäureanhydrid und nachfolgender Reaktion mit Glycidylmethacrylat der 2-Methacroylethyl, 3-Methacroyl-2-hydroxypropylester der 1,2-Cyclohexancarbonsäure

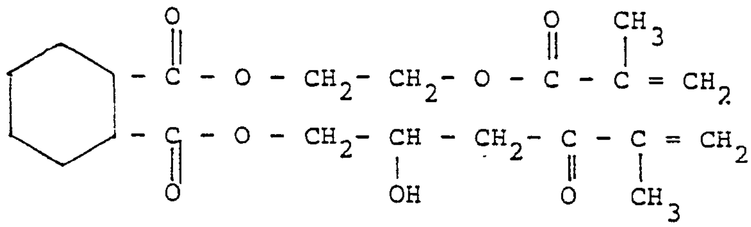

hergestellt.

b) Das unter a) erhaltene Reaktionsprodukt wurde, wie in den vorhergehenden Beispielen beschrieben, mit 1,6-Hexamethylendiisocyanat zum entsprechenden Hexamethylenbiscarbamat der Formel

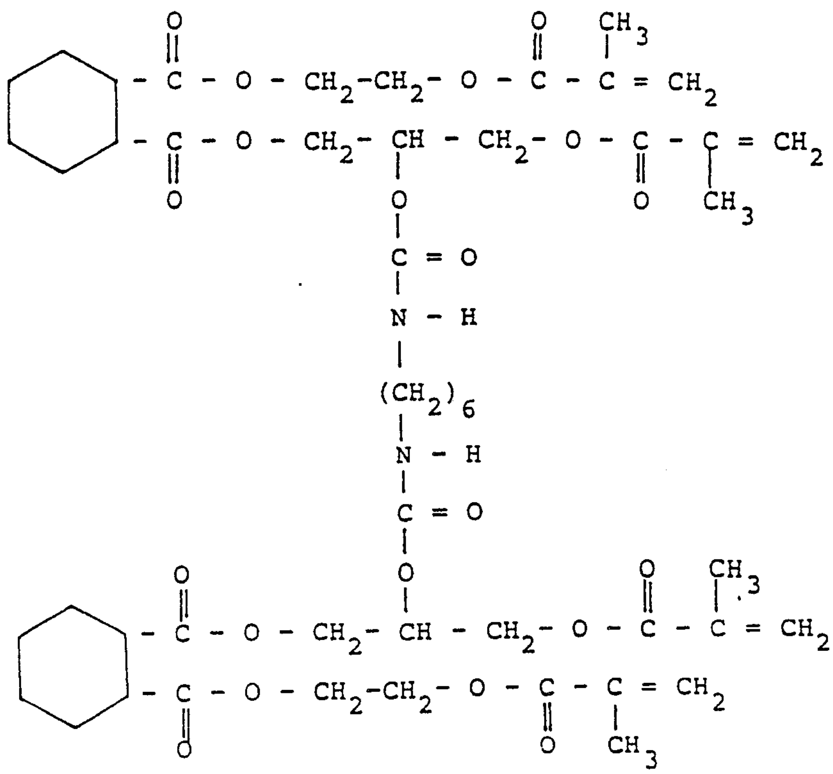

umgesetzt.

Auf analoge Weise ließen sich das entsprechende 1,4-Tetramethylenbiscarbamat und das Methylenbisphenyl- bzw. -biscyclohexylcarbamat herstellen.

Die folgenden Beispiele dokumentieren die überlegenen Eigenschaften der durch Mitverwendung der erfindungsgemäßen Monomeren hergestellten dentalen Restorationsmaterialien.

Beispiel 1

| | Teil A | Teil B |
|---|---|---|
| | (Gewichtsteile) | |
| 2,2-Bis [4'(2"-methacroylethoxy)phenyl]propan | 56 | 56 |
| 2,2-Bis [4'(3"-methacroyl-2"-hydroxypropoxy)phenyl]propan | 10 | 10 |

(Fortsetzung)

| | Teil A | Teil B |
|---|---|---|
| | (Gewichtsteile) | |
| Triethylenglykoldimethacrylat | 19 | 19 |
| Hexamethylenbiscarbamat des 2-Methacroyl-, 3-Methacroyl-2-hydroxypropyl(phthalates) | 15 | 15 |
| Silanisiertes Bariumborosilikatglas (Mittl. Teilchendurchmesser ~10 Mikrons) | 600 | 600 |
| Silanisierte Kieselsäure (Teilchendurchmesser~25- 50 Millimikrons) | 40 | 40 |
| 2,6-Di-tert.-butyl-4-methylphenol | 0,03 | 0,12 |
| 2-Hydroxy-4-methoxybenzophenon | 0,6 | 0,6 |
| Benzoylperoxid | | 2 |
| N,N-Bis(hydroxyethyl)-p-toluidin | 4 | |

Zur Aushärtung werden gleiche Mengen von A und B gemischt und bei 23 °C 150 Sekunden auspolymerisiert.

Das erhaltene Polymerisat wies folgende Eigenschaften auf :

| | |
|---|---|
| Transluzenz-Faktor ($C_{70}$) : | 0,4 |
| Diametrale Zugfestigkeit : | 7 500 psi |
| Wasserabsorption : | 0,42 mg/cm$^2$ |
| Härte (Barcol) : | 98 |

Beispiel 2

| | Teil A | Teil B |
|---|---|---|
| | (Gewichtsteile) | |
| 2,2-Bis 4'(2"-methacroylethyloxy) phenyl propan | 56 | 56 |
| 2,2-Bis 4'(3"-methacroyl-2"-hydroxypropoxy)phenyl propan | 10 | 10 |
| Triethylenglykoldimethacrylat | 19 | 19 |
| 2-Hydroxy-4-methoxybenzophenon | 0,6 | 0,6 |
| 2,6-Di-tert.-butyl-4-methylphenol | 0,03 | 0,12 |
| N,N-bis(diethanol)-p-toluidin | 4 | |
| Benzoylperoxid | | 2 |
| Hexamethylenbiscarbamat des 2-Methacroylethyl-, 3-Methacroyl-2-hydroxypropyl(maleats) | 15 | 15 |

(Fortsetzung)

| | Teil A | Teil B |
|---|---|---|
| | (Gewichtsteile) | |
| Silanisiertes Bariumborosilikatglas (Teilchengröße 10 Mikrons) | 600 | 600 |
| Silanisierte Kieselsäure (Teilchengröße 25 - 50 Millimikrons) | 40 | 40 |

Zur Aushärtung werden gleiche Mengen von A und B gemischt und bei 23 °C 150 Sekunden auspolymerisiert.

Das erhaltene Polymerisat wies folgende Eigenschaften auf :

| | |
|---|---|
| Transluzenz-Faktor ($C_{70}$) : | 0,4 |
| Diametrale Zugfestigkeit : | 5 700 psi |
| Wasserabsorption : | 0,59 mg/cm$^2$ |
| Härte (Barcol) : | 97 |

Beispiel 3

| | Teil A | Teil B |
|---|---|---|
| | (Gewichtsteile) | |
| 2,2-Bis 4'(2"-methacroylethyloxy) phenyl propan | 56 | 56 |
| 2,2-Bis 4'(3"-methacroyl-2"-hydroxy-propoxy)phenyl propan | 10 | 10 |
| Triethylenglykoldimethacrylat | 19 | 19 |
| 2-Hydroxy-4-methoxybenzophenon | 0,6 | 0,6 |
| 2,6-Di-tert.-butyl-4-methylphenol | 0,03 | 0,12 |
| N,N-Bis(hydroxyethyl)-p-toluidin | 4 | |
| Benzoylperoxid | | 2 |
| Hexamethylenbiscarbamat des 2-Methacroylethyl-, 3-Methacroyl-2-hydroxypropylesters der Cyclohexan-1,2-dicarbonsäure | 15 | 15 |
| Silanisiertes Bariumborosilikatglas (Teilchengröße 5 - 15 Mikrons) | 600 | 600 |
| Silanisierte Kieselsäure (Teilchengröße 20 - 50 Millimikrons) | 40 | 40 |

Zur Aushärtung werden gleiche Mengen von A und B gemischt und bei 23 °C 150 Sekunden auspolymerisiert.

Das erhaltene Polymerisat wies folgende Eigenschaften auf :

| | |
|---|---|
| Transluzenz-Faktor ($C_{70}$) : | 0,4 |
| Diametrale Zugfestigkeit : | 6 200 psi |
| Wasserabsorption : | 0,42 mg/cm² |
| Härte (Barcol) : | 98 |

Beispiel 4

| | Teil A | Teil B |
|---|---|---|
| | (Gewichtsteile) | |
| 2,2-Bis 4'(2"-methacroylethyloxy) phenyl propan | -- | 56 |
| 2,2-Bis 4'(3"-methacroyl-2"-hydroxy-propoxy)phenyl propan | -- | 10 |
| Triethylenglykoldimethacrylat | 20 | 19 |
| Methylenbisphenylcarbamat des 2-Methacroylethyl-, 3-Methacroyl-2-hydroxy-propyl(hexahydrophthalats) | 80 | 15 |
| 2-Hydroxy-4-methoxybenzophenon | 0,5 | 0,5 |
| 2,6-Di-tert.-butyl-4-methylphenol | 0,03 | 0,12 |
| N,N-Bis(hydroxyethyl)-p-toluidin | 4 | |
| Benzoylperoxid | | 2,2 |
| Silanisiertes Bariumborosilikatglas (Teilchengröße 5- 15 Mikrons) | 600 | 600 |
| Silanisierte Kieselsäure (Teilchengröße 25 - 50 Millimikrons) | 40 | 40 |

Zur Aushärtung werden gleiche Mengen von A und B gemischt und bei 23 °C 150 Sekunden auspolymerisiert.

Das erhaltene Polymerisat wies folgende Eigenschaften auf :

| | |
|---|---|
| Transluzenz-Faktor ($C_{70}$) : | 0,45 |
| Diametrale Zugfestigkeit : | 8 200 psi |
| Wasserabsorption : | 0,65 mg/cm² |
| Härte (Barcol) : | 98 |

Im folgenden wird noch ein Beispiel für einen UV-härtenden Dentallack gegeben :

| | |
|---|---|
| Hexamethylenbiscarbamat des 2-Methacroylethyl-, 3-Methacroyl-2-hydroxypropylesters der Cis-Norbornendicarbonsäure (Bicyclo 2.2.1. hept-2-en-2,3-dicarbonsäure) | 12,0 Gew.-% |
| 2-Ethylhexylmethacrylat | 23,0 Gew.-% |
| Ethylenglykoldimethacrylät | 25,0 Gew.-% |
| 1,6-Hexandioldimethacrylat | 28,3 Gew.-% |
| Acetophenon | 0,7 Gew.-% |
| N,N-Di(2-hydroxyethyl)-p-toluidin | 1,0 Gew.-% |

**Patentansprüche** (für die Vertragsstaaten : BE, CH, DE, FR, GB, IT, LI, NL, SE)

1. Neue Tetra(meth)acrylsäureverbindungen der allgemeinen Formel

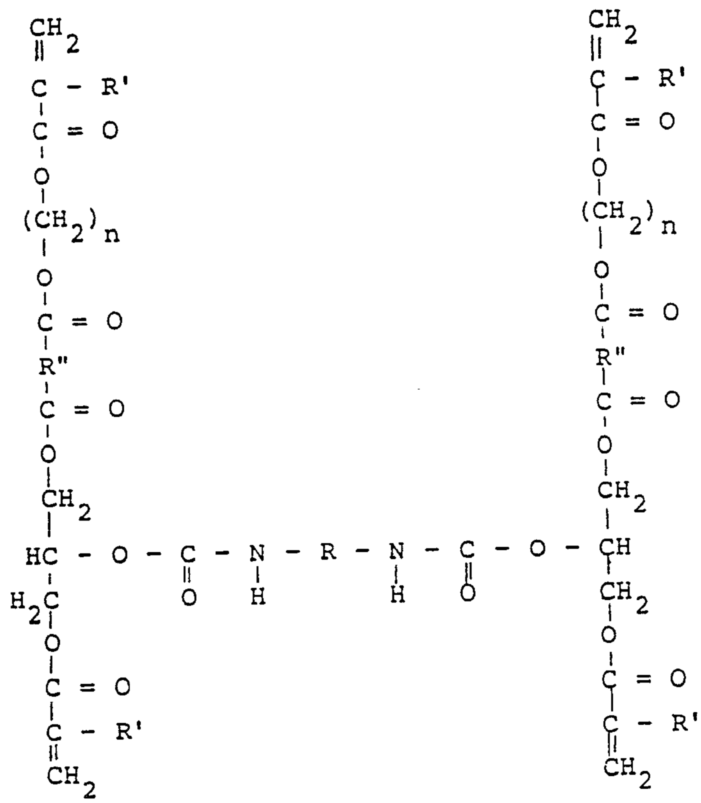

wobei R einen zweiwertigen (ar)aliphatischen, cycloaliphatischen, oder aromatischen Rest mit 4 bis 18 C-Atomen, R′ H o. Methyl, R″ einen —CH=CH—, $—CH_2CH_2—$, $—CH_2CH_2CH_2—$, $—CH_2CH_2CH_2CH_2—$, ggf. substituierten bzw. hydrierten Benzol- oder einen Cyclohexan- oder einen Cis-Norbornenrest, und n 2 oder 3 bedeuten.

2. Verbindungen der allgemeinen Formel nach Anspruch 1, dadurch gekennzeichnet, daß R einen Hexamethylenrest bedeutet.

3. Verbindungen der allgemeinen Formel nach Anspruch 1, dadurch gekennzeichnet, daß R einen Phenylen- oder Toluylenrest bedeutet.

4. Verbindungen der allgemeinen Formel nach Anspruch 1, dadurch gekennzeichnet, daß R einen Methylenbiscyclohexylrest bedeutet.

5. Verbindungen der allgemeinen Formel nach Anspruch 1, dadurch gekennzeichnet, daß R einen Methylenbisphenylrest bedeutet.

6. Verbindungen der allgemeinen Formel nach Anspruch 1, dadurch gekennzeichnet, daß R einen Tetramethylenrest bedeutet.

7. Verbindungen der allgemeinen Formel nach einem der Ansprüche 1 bis 7, dadurch gekennzeichnet, daß R″ eine —CH=CH-Gruppe bedeutet.

8. Verbindungen der allgemeinen Formel nach einem der Ansprüche 1 bis 7, dadurch gekennzeichnet, daß R″ einen Benzolrest bedeutet.

9. Verbindungen der allgemeinen Formel nach einem der Ansprüche 1 bis 7, dadurch gekennzeichnet, daß R″ einen Tetrahydrobenzolrest bedeutet.

10. Verbindungen der allgemeinen Formel nach einem der Ansprüche 1 bis 7, dadurch gekennzeichnet, daß R″ einen Hexahydrobenzolrest bedeutet.

11. Verbindungen der allgemeinen Formel nach einem der Ansprüche 1 bis 7, dadurch gekennzeichnet, daß R″ einen Cyclohexanrest bedeutet.

12. Verwendung einer oder mehrerer Verbindungen nach den Ansprüchen 1 bis 11 als Bindemittel.

13. Verwendung einer oder mehrerer Verbindungen nach den Ansprüchen 1 bis 11 als Bindemittel in dentalen Behandlungsmaterialien.

14. Dentales Restorations- und Füllungsmaterial, dadurch gekennzeichnet, daß es neben Füllstoffen, Initiatoren, Beschleunigern und weiteren üblichen Bestandteilen mindestens eine Verbindung nach den Ansprüchen 1 bis 11 enthält.

15. Dentales Restorations- und Füllungsmaterial nach Anspruch 14, dadurch gekennzeichnet, daß es die folgenden Bestandteile enthält :

5 bis 50 Gew.-% mindestens einer Verbindung nach den Ansprüchen 1 bis 11 ;

0,1 bis 5,0 Gew.-% mindestens eines Polymerisationsinitiators und/oder Polymerisationsbeschleunigers ;

50 bis 90 Gew.-% mindestens eines Füllstoffs ;

sowie gegebenenfalls weitere polymerisierbare Verbindungen, UV-Absorber, Farbstoffe, Haftungsverbesserer und sonstige in solchen Mitteln an sich übliche Zusätze.

**Patentansprüche** (für den Vertragsstaat AT)

1. Verfahren zur Herstellung von neuen Tetra(meth)acrylsäureverbindungen der allgemeinen Formel

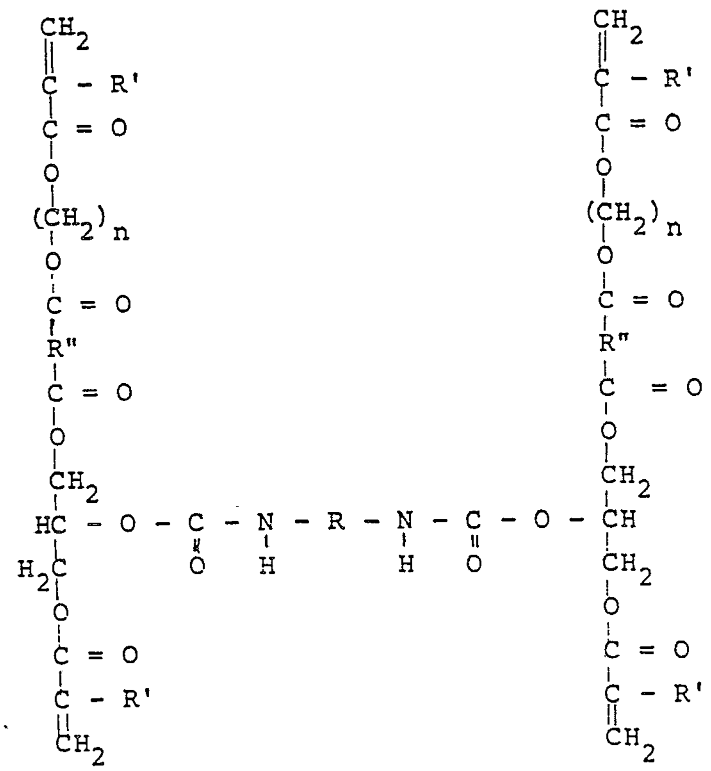

wobei R einen zweiwertigen (ar)aliphatischen, cycloaliphatischen, oder aromatischen Rest mit 4 bis 18 C-Atomen, R' H o. Methyl, R" einen —CH=CH—, —$CH_2CH_2$—, —$CH_2CH_2CH_2$—, —$CH_2CH_2CH_2CH_2$—, ggf. substituierten bzw. hydrierten Benzol- oder einen Cyclohexan- oder einen Cis-Norbornenrest, und n 2 oder 3 bedeuten, dadurch gekennzeichnet, daß man Verbindungen der allgemeinen Formel

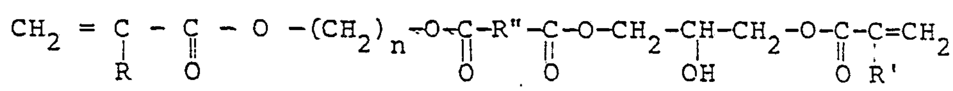

mit Diisocyanaten der allgemeinen Formel

O=C=N—R—N=C=O,

wobei R, R', R" und n die oben angegebene Bedeutung haben, umsetzt.

2. Verfahren nach Anspruch 1, dadurch gekennzeichnet, daß R einen Hexamethylenrest bedeutet.

3. Verfahren nach Anspruch 1, dadurch gekennzeichnet, daß R einen Phenylen- oder Toluylenrest bedeutet.

4. Verfahren nach Anspruch 1, dadurch gekennzeichnet, daß R einen Methylenbiscyclohexylrest bedeutet.

5. Verfahren nach Anspruch 1, dadurch gekennzeichnet, daß R einen Methylenbisphenylrest bedeutet.

6. Verfahren nach Anspruch 1, dadurch gekennzeichnet, daß R einen Tetramethylenrest bedeutet.

7. Verfahren nach einem der Ansprüche 1 bis 7, dadurch gekennzeichnet, daß R" eine —CH=CH-Gruppe bedeutet.

8. Verfahren nach einem der Ansprüche 1 bis 7, dadurch gekennzeichnet, daß R″ einen Benzolrest bedeutet.

9. Verfahren nach einem der Ansprüche 1 bis 7, dadurch gekennzeichnet, daß R″ einen Tetrahydrobenzolrest bedeutet.

10. Verfahren nach einem der Ansprüche 1 bis 7, dadurch gekennzeichnet, daß R″ einen Hexahydrobenzolrest bedeutet.

11. Verfahren nach einem der Ansprüche 1 bis 7, dadurch gekennzeichnet, daß R″ einen Cyclohexanrest bedeutet.

12. Verwendung einer oder mehrerer nach den Ansprüchen 1 bis 11 hergestellten Verbindungen als Bindemittel.

13. Verwendung einer oder mehrerer nach den Ansprüchen 1 bis 11 hergestellten Verbindungen als Bindemittel in dentalen Behandlungsmaterialien.

14. Dentales Restorations- und Füllungsmaterial, dadurch gekennzeichnet, daß es neben Füllstoffen, Initiatoren, Beschleunigern und weiteren üblichen Bestandteilen mindestens eine nach den Ansprüchen 1 bis 11 hergestellte Verbindung enthält.

15. Dentales Restorations- und Füllungsmaterial nach Anspruch 14, dadurch gekennzeichnet, daß es die folgenden Bestandteile enthält :

5 bis 50 Gew.-% mindestens einer nach den Ansprüchen 1 bis 11 hergestellten Verbindung ;

0,1 bis 5,0 Gew.-% mindestens eines Polymerisationsinitiators und/oder Polymerisationsbeschleunigers ;

50 bis 90 Gew.-% mindestens eines Füllstoffs ;

sowie gegebenenfalls weitere polymerisierbare Verbindungen, UV-Absorber, Farbstoffe, Haftungsverbesserer und sonstige in solchen Mitteln an sich übliche Zusätze.

**Claims** (for the Contracting States : BE, CH, DE, FR, GB, IT, LI, NL)

1. New tetraacrylic and tetramethacrylic acid esters of the general formula

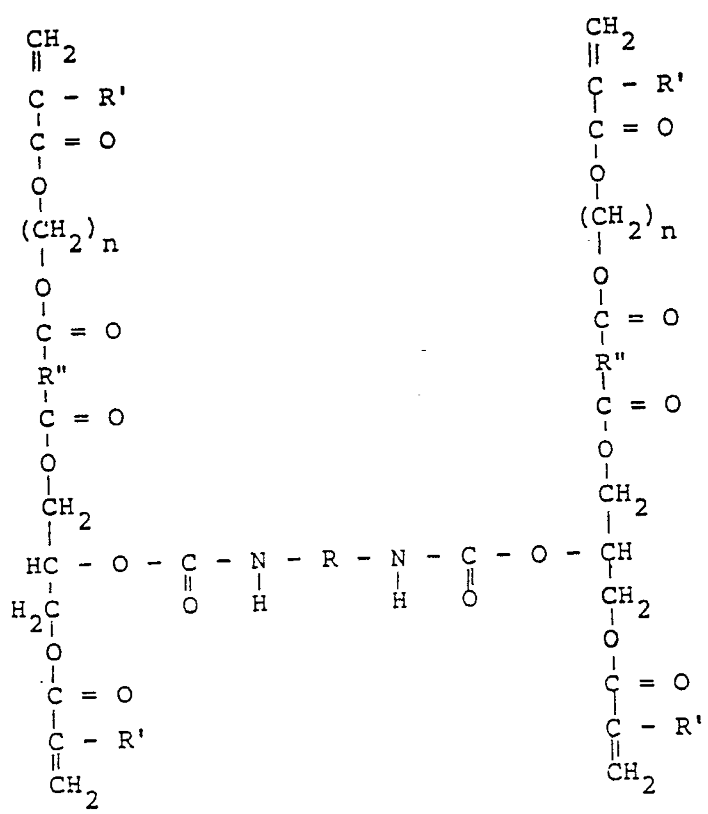

where R is a divalent (ar)aliphatic, cycloaliphatic, or aromatic group with 4 to 18 carbon atoms ; R′ is H or methyl ; R″ is a —CH=CH—, $—CH_2CH_2—$, $—CH_2CH_2CH_2—$, $—CH_2CH_2CH_2CH_2—$, an optionally substituted or hydrogenated benzene group, a cyclohexane group, or a cis-norbornene group, and n is 2 or 3.

2. Compounds according to the general formula of claim 1, where R is a hexamethylene group.

3. Compounds according to the formula of claim 1, where R is a phenylene or toluylene group.

4. Compounds according to the formula of claim 1, where R is a methylene biscyclohexyl group.

5. Compounds according to the formula of claim 1, where R is a methylene bisphenyl group.

6. Compounds according to the formula of claim 1, where R is a tetramethylene group.

7. Compounds according to the formula of one of the claims 1 to 7, where R″ is a —CH=CH-group.

8. Compounds according to the formula of one of the claims 1 to 7, where R″ is a benzene group.

9. Compounds according to the formula of one of the claims 1 to 7, where R″ is a tetrahydrobenzene group.

10. Compounds according to the formula of one of the claims 1 to 7, where R″ is a hydrobenzene group.

11. Compounds according to the formula of one of the claims 1 to 7, where R″ is a cyclohexane group.

12. Use of one or more of the compounds according to the claims 1 to 11 as binding agent.

13. Use of one or more compounds according to the claims 1 to 11 as binding agent in dental materials.

14. Dental restorative and filling material containing at least one inorganic filler, at least one polymerization initiator, at least one polymerization accelerator, and optionally other components common in such compositions, containing at least one of the compounds according to the claims 1 to 11.

15. Dental restorative and filling material according to claim 14, containing the following components :

5 to 50 % by weight of at least one of the compounds according to the claims 1 to 11 ;

0,1 bis 5,0 % by weight of at least one polymerization initiator and/or polymerization accelerator ;

50 to 90 % by weight of at least one filler ;

optionally additional polymerizable compounds, UV-absorbers, dyes, adhesion improvers, and additional components common in those preparations.

**Claims** (for the Contracting State AT)

1. Preparation of new tetraacrylic and tetramethacrylic acid esters of the general formula

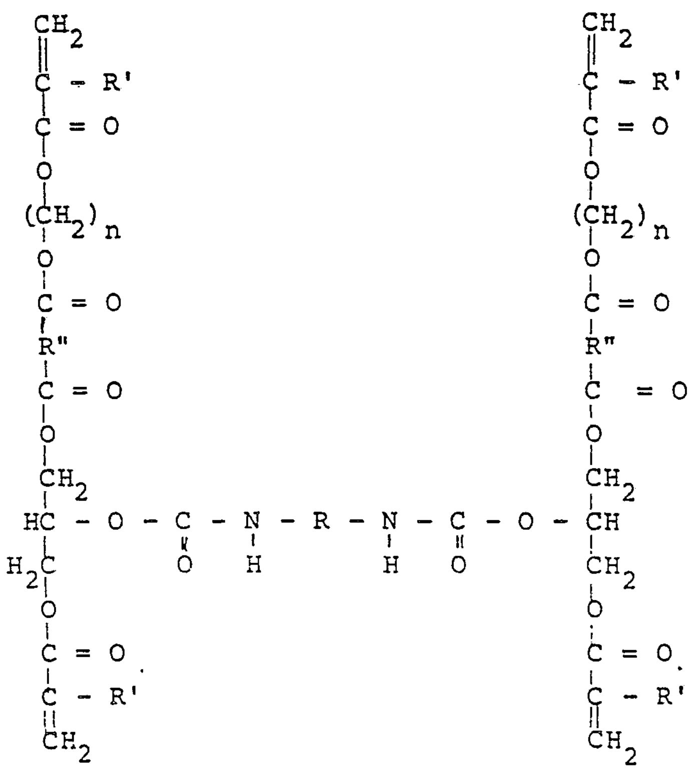

where R is a divalent (ar)aliphatic, cycloaliphatic, or aromatic group with 4 to 18 carbon atoms ; R′ is H or methyl ; R″ is a —CH=CH—, $—CH_2CH_2—$, $—CH_2CH_2CH_2—$, $—CH_2CH_2CH_2CH_2—$, an optionally substituted or hydrogenated benzene group, a cyclohexane group, or a cis-norbornene group, and n is 2 or 3 ; characterized in that compounds of the general formula

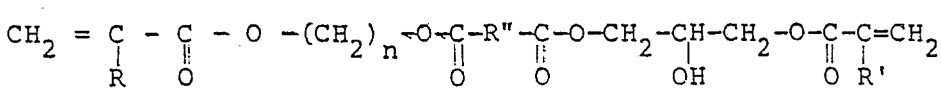

are reacted with diisocyanates of the general formula

$$O{=}C{=}N{-}R{-}N{=}C{=}O\,.$$

2. Preparation according to the general formula of claim 1, where R is a hexamethylene group.

3. Preparation according to the formula of claim 1, where R is a phenylene or toluylene group.

4. Preparation according to the formula of claim 1, where R is a methylene biscyclohexyl group.

5. Preparation according to the formula of claim 1, where R is a methylene bisphenyl group.

6. Preparation according to the formula of claim 1, where R is a tetramethylene group.

7. Preparation according to the formula of one of the claims 1 to 7, where R″ is a —CH=CH-group.

8. Preparation according to the formula of one of the claims 1 to 7, where R″ is a benzene group.

9. Preparation according to the formula of one of the claims 1 to 7, where R″ is a tetrahydrobenzene group.

10. Preparation according to the formula of one of the claims 1 to 7, where R″ is a hexahydrobenzene group.

11. Preparation according to the formula of one of the claims 1 to 7, where R″ is a cyclohexane group.

12. Use of one or more of the compounds prepared according to the claims 1 to 11 as binding agent.

13. Use of one or more compounds prepared according to the claims 1 to 11 as binding agent in dental materials.

14. Dental restorative and filling material containing at least one inorganic filler, at least one polymerization initiator, at least one polymerization accelerator, and optionally other components common in such compositions, containing at least one of the compounds according to the claims 1 to 11.

15. Dental restorative and filling material according to claim 14, containing the following components :

5 to 50 % by weight of at least one of the compounds according to the claims 1 to 10 ;

0,1 to 5,0 % by weight of at least one polymerization initiator and/or polymerization accelerator ;

50 to 90 % by weight of at least one filler ;

optionally additional polymerizable compounds, UV-absorbers, dyes, adhesion improvers, and additional components common in those preparations.

**Revendications** (pour les Etats contractants : BE, CH, DE, FR, GB, IT, LI, NL)

1. Nouveaux composés ayant quatre groupements d'acide acrylique ou méthacrylique répondant à la formule générale

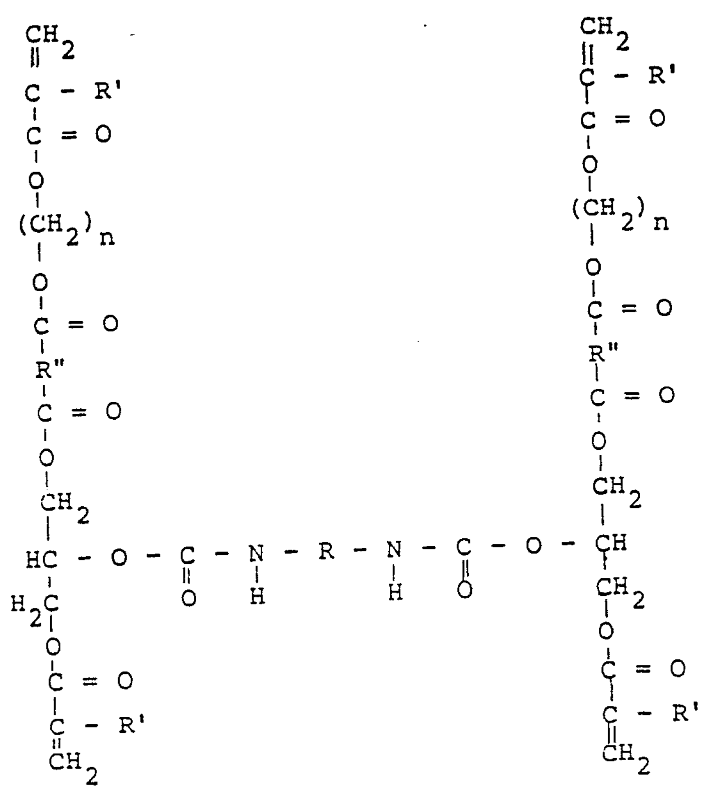

dans laquelle R représente un radical divalent aliphatique, araliphatique, cycloaliphatique ou aromatique, ayant de 4 à 18 atomes de carbone, R′ représente un atome de H ou un radical méthyle, R″ représente un

groupe $—CH{=}CH—$, $—CH_2CH_2—$, $—CH_2CH_2CH_2—$, $—CH_2CH_2CH_2CH_2—$, un radical de benzène éventuellement substitué ou respectivement hydrogéné ou un radical de cyclohexane ou de cis-norbornène, et n est égal à 2 ou à 3.

2. Composés répondant à la formule générale de la revendication 1, caractérisés en ce que R représente un radical d'hexaméthylène.

3. Composés répondant à la formule générale de la revendication 1, caractérisés en ce que R représente un radical de phénylène ou de toluylène.

4. Composés répondant à la formule générale de la revendication 1, caractérisés en ce que R représente un radical de méthylènebiscyclohexyle.

5. Composés répondant à la formule générale de la revendication 1, caractérisés en ce que R représente un radical de méthylènebisphényle.

6. Composés répondant à la formule générale de la revendication 1, caractérisés en ce que R représente un radical de tétraméthylène.

7. Composés de la formule générale, suivant l'une des revendications 1 à 7, caractérisés en ce que R″ représente un groupe $—CH{=}CH—$.

8. Composés de la formule générale, suivant l'une des revendications 1 à 7, caractérisés en ce que R″ représente un radical de benzène.

9. Composés de la formule générale, suivant l'une des revendications 1 à 7, caractérisés en ce que R″ représente un radical de tétrahydrobenzène.

10. Composés de la formule générale, suivant l'une des revendications 1 à 7, caractérisés en ce que R″ représente un radical d'hexahydrobenzène.

11. Composés de la formule générale, suivant l'une des revendications 1 à 7, caractérisés en ce que R″ représente un radical de cyclohexane.

12. Utilisation d'un ou de plusieurs composés suivant les revendications 1 à 11, comme agent liant.

13. Utilisation d'un ou de plusieurs composés suivant les revendications 1 à 11, comme agent liant dans des matières de traitement dentaire.

14. Matière d'obturation et de restauration dentaire, caractérisée en ce qu'elle contient, outre des substances de remplissage, des initiateurs, des accélérateurs et d'autres composants courants, au moins un composé suivant les revendications 1 à 11.

15. Matière d'obturation et de restauration dentaire suivant la revendication 14, caractérisée en ce qu'elle contient les composants suivants :

5 à 50 % en poids d'au moins un composé suivant les revendications 1 à 11 ;

0,1 à 5,0 % en poids d'au moins un initiateur de polymérisation et/ou accélérateur de polymérisation ;

50 à 90 % en poids d'au moins une substance de remplissage,

ainsi qu'éventuellement d'autres composés polymérisables, des agents d'absorbtion pour les UV, des colorants, des agents améliorant l'adhésion et d'autres additifs courants en soi dans de tels produits.

**Revendications** (pour l'Etat contractant AT)

1. Procédé de préparation de nouveaux composés ayant quatre groupements d'acide acrylique ou méthacrylique, qui répondent à la formule générale

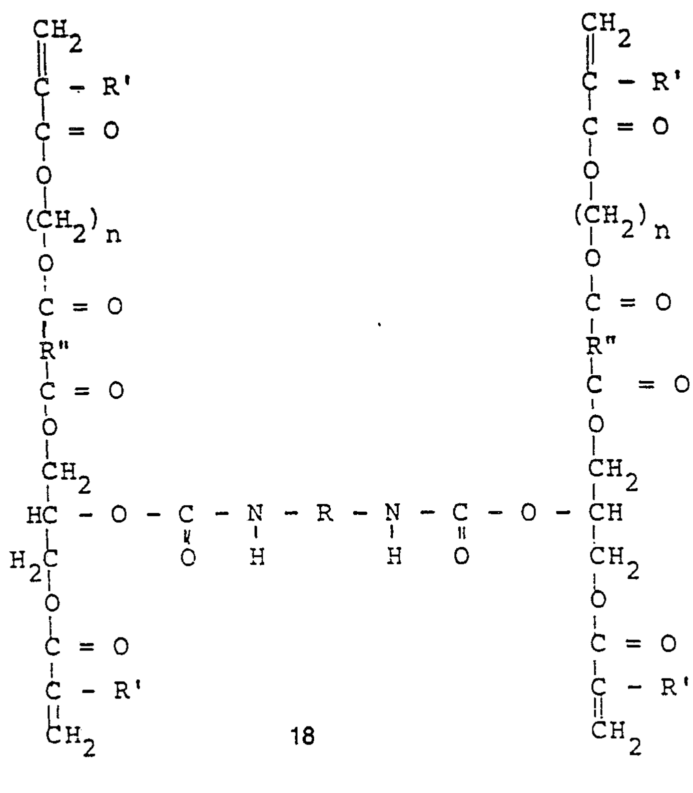

dans laquelle R représente un radical divalent aliphatique, araliphatique, cycloaliphatique ou aromatique, ayant de 4 à 18 atomes de carbone, R′ représente un atome de H ou un radical méthyle, R″ représente un groupe $-CH=CH-$, $-CH_2CH_2-$, $-CH_2CH_2CH_2-$, $-CH_2CH_2CH_2CH_2-$, un radical de benzène éventuellement substitué ou respectivement hydrogéné ou un radical de cyclohexane ou de cis-norbornène, et n est égal à 2 ou à 3, caractérisé en ce qu'on fait réagir des composés de la formule générale

$$CH_2=\underset{R}{\underset{|}{C}}-\underset{O}{\underset{\|}{C}}-O-(CH_2)_n-O-\underset{O}{\underset{\|}{C}}-R''-\underset{O}{\underset{\|}{C}}-O-CH_2-\underset{OH}{\underset{|}{CH}}-CH_2-O-\underset{O}{\underset{\|}{C}}-\underset{R'}{\underset{|}{C}}=CH_2$$

avec des diisocyanates de la formule générale

$$O=C=N-R-N=C=O\,,$$

où R, R′, R″ et n ont la même signification que celle donnée ci-dessus.

2. Procédé suivant la revendication 1, caractérisé en ce que R représente un radical d'hexaméthylène.

3. Procédé suivant la revendication 1, caractérisé en ce que R représente un radical de phénylène ou de toluylène.

4. Procédé suivant la revendication 1, caractérisé en ce que R représente un radical de méthylènebiscyclohexyle.

5. Procédé suivant la revendication 1, caractérisé en ce que R représente un radical de méthylènebisphényle.

6. Procédé suivant la revendication 1, caractérisé en ce que R représente un radical de tétraméthylène.

7. Procédé suivant l'une des revendications 1 à 7, caractérisé en ce que R″ représente un groupe $-CH=CH-$.

8. Procédé suivant l'une des revendications 1 à 7, caractérisé en ce que R″ représente un radical de benzène.

9. Procédé suivant l'une des revendications 1 à 7, caractérisé en ce que R″ représente un radical de tétrahydrobenzène.

10. Procédé suivant l'une des revendications 1 à 7, caractérisé en ce que R″ représente un radical d'hexahydrobenzène.

11. Procédé suivant l'une des revendications 1 à 7, caractérisé en ce que R″ représente un radical de cyclohexane.

12. Utilisation d'un ou de plusieurs composés préparés selon les revendications 1 à 11, comme agent liant.

13. Utilisation d'un ou de plusieurs des composés préparés selon les revendications 1 à 11, comme agent liant dans des matières de traitement dentaire.

14. Matière d'obturation et de restauration dentaire, caractérisée en ce qu'elle contient, outre des substances de remplissage, des initiateurs, des accélérateurs et d'autres composants courants, au moins un composé préparé selon les revendications 1 à 11.

15. Matière d'obturation et de restauration dentaire suivant la revendication 14, caractérisée en ce qu'elle contient les composants suivants :

5 à 50 % en poids d'au moins un composé préparé selon les revendications 1 à 11 ;

0,1 à 5,0 % en poids d'au moins un initiateur de polymérisation et/ou accélérateur de polymérisation ;

50 à 90 % en poids d'au moins une substance de remplissage ;

ainsi qu'éventuellement d'autres composés polymérisables, des agents d'absorbtion pour les UV, des colorants, des agents améliorant l'adhésion et d'autres additifs courants en soi dans de tels produits.